# EUROPEAN PATENT APPLICATION

(11) **EP 1 974 626 A1**
(43) Date of publication of application: **01.10.2008**
(21) Application number: 07105124.7
(22) Date of filing: 28.03.2007
(51) Int. Cl.: A43B 7/20, A43B 13/28, A61F 5/01, A61F 13/08

(54) **Legging**

(71) Applicant: CAMP SCANDINAVIA AB, 254 67 Helsingborg (SE)
(72) Inventor: Allard, Peter, 254 40 Helsingborg (SE)
(74) Representative: Akerman, Marten Lennart

(57) **Abstract**

The invention relates to a legging and particularly a legging (1) provided with holding means for an insole (5) improving the function of the legging when used without an outer rigid shell. The legging is suitable for providing compression on a leg and foot, the legging (1) being adapted to substantially cover the leg and foot, characterised by a holding means (6a, 6b) for securing a support member (5) at the plantar side of the foot. In embodiments of the invention, the holding means comprises pockets, suitably a front pocket (6a) and a rear pocket (6b), or two side pockets, or straps.

## Description

### Field of the invention

The present invention relates to a legging and particularly a compression legging provided with holding means for an insole improving the function of the legging when used without an outer rigid shell.

### Background of the invention

To improve foot wound healing, e.g. on patients having diabetes, it is known to provide compression around the lower leg, since this will increase the blood circulation through the wound.

It is not possible to use commercially available pressure stockings, since these have to be pulled over the foot, which can adversely affect the wound. It is therefore necessary to construct leggings, which can easily be taken on and off. Providing them with zip fasteners can take care of this need.

US 2003/0195449 and US 7,037,282 show examples of medical support appliances provided with a single zip lock. The appliances have a fixed circumference and have to be custom made for each patient to provide optimal effect. Thus, there is a need for an off-the-shelf item with controllable compression that does not have to be tailored to each patient.

Some leggings are designed to be used with an outer rigid shell providing additional rigidity and assisting the compressing function of the legging. However, patients tend to take off the shell for comfort when lying or sitting and not put the shell back on when walking short distances. Thus, there is a need for a support under the foot even when the legging is not used with the shell.

US 6,393,734 discloses a foot care article comprising a protective insole and cover bonded together. However, this is not a compression legging and the insole is not removable.

### Summary of the invention

An object of the present invention is to provide a legging with holding means for carrying a support member providing support for the foot even without any other stabilising structure.

The present invention provides a legging for a leg and foot, the legging being adapted to substantially cover the leg and foot, characterised by a holding means for securing a support member at the plantar side of the foot.

In embodiments of the invention, the holding means comprises pockets, suitably a front pocket and a rear pocket, or two side pockets.

In other embodiments of the invention, the holding means comprises straps.

The invention is defined in the appended claim 1, while embodiments are set forth in the dependent claims.

### Brief description of the drawings

The invention will be described in detail below with reference to the accompanying drawings, in which:
Fig. 1 is a perspective side view of an embodiment of a legging according to the invention,
Fig. 2 is a bottom view of a holding means comprising a compartment without insole according to one embodiment of the invention,
Fig. 3 is a bottom view of the compartment with inserted insole,
Fig. 4 is a bottom view of the compartment with partly inserted insole,
Fig. 5 is a bottom view of a holding means comprising a compartment according to a further embodiment of the invention,
Fig. 6 is a bottom view of a holding means comprising a compartment according to a another embodiment of the invention,
Fig. 7 is a bottom view of a holding means comprising straps according to one embodiment of the invention,
Fig. 8 is a bottom view of a holding means comprising straps according to another embodiment of the invention,
Fig. 9 is a perspective view of an Achilles portion of an embodiment of the invention, and
Fig. 10 is a perspective view of a legging inserted in a shell according to one embodiment of the invention.

### Detailed description of preferred embodiments

The invention generally relates to a medical support appliance in the form of a legging, particularly a compression legging for treating foot wounds. Several studies have shown that it is positive for patients suffering from ulcers and/or oedema to use compression stockings or other equipment giving the same effect. The reason is that the increased pressure stimulates the blood flow through the wound. The increased pressure (15-20 mm Hg) around the leg can be achieved with a bandage, but this takes rather long time to apply and requires a skilled person.

It is not possible to use traditional pressure stockings, since these have to be pulled over the foot, which can adversely affect to wound and relocate dressings or the like on the foot. It is therefore necessary to have a product which can easily be taken on and off.

Orthoses comprising an inner legging and outer rigid shell have been used with good results. One problem is that the outer shell is cumbersome and often taken off for greater comfort. Some orthoses rely on the shell to provide adequate compression and thus the effect is lost. Thus, the legging should by itself be able to provide a large degree of compression. In an embodiment, the legging essentially is manufactured of a stretchable material.

In one embodiment, the material is a so-called spacer fabric. A spacer fabric is principally a composite material, consisting of three material layers: a first outer layer, a second outer layer, and an intermediate layer comprising pile yarns or fibres, integrated with and interconnecting the two outer layers. A spacer fabric is manufactured in one knitted process, without the need for lamination or other adhesive means.

Woven as well as knitted spacer fabrics exist, and both weft knitted and warp knitted spacers exists. Recently, also so-called spacer fabrics based on non-woven technology have been developed.

In a suitable material, polyester yarns are knitted together with elastane, to form an elastic spacer fabric with good cushioning and stretch/recovery properties, suitable for orthopaedic end uses. This fabric is a weft knitted spacer fabric, knitted on a circular knitting machine.

Since it should be possible to take on and off the legging without pulling, it should be possible to open the legging. A zip lock can provide this function.

For best function the orthosis should also comprise an insole or other support member to distribute the pressure under the foot in a supportive and comfortable way. According to embodiments of the present invention, the support member is removably attached to the legging, not to the outer shell. Thus, the support will remain when the shell is taken off. The insole or support member should be removable so that it can be tailor-made for the patient or provided in left or right versions etc. In a basic form the insole may be flat and fit both a right and a left foot.

The invention is also applicable to leggings only intended for support, with moderate or zero compression, or with the primary function of just carrying an insole.

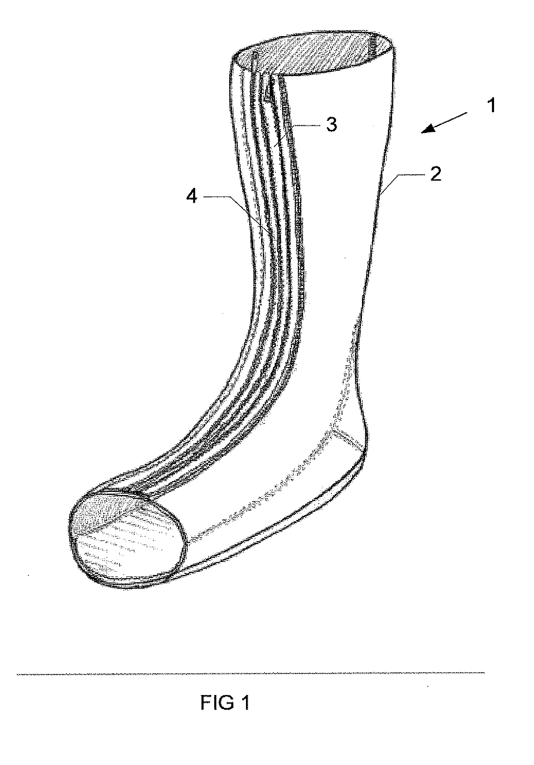

Fig. 1 is a perspective view of an embodiment of a legging 1 according to the invention. The legging 1 may comprise a main part 2 substantially covering the leg, typically the lower leg and the foot. The legging is attached to the leg by means of a fastening means 4, here shown in the form of zip fasteners, provided on the main part and on a tongue 3. The tongue 3 is arranged at the inside of the legging 1 such that the fastening means 4 do not come into contact with the leg. The fastening means 4 provide an adjustability of the circumference. The fastening means and adjustability of the legging 1 do not form part of the present invention.

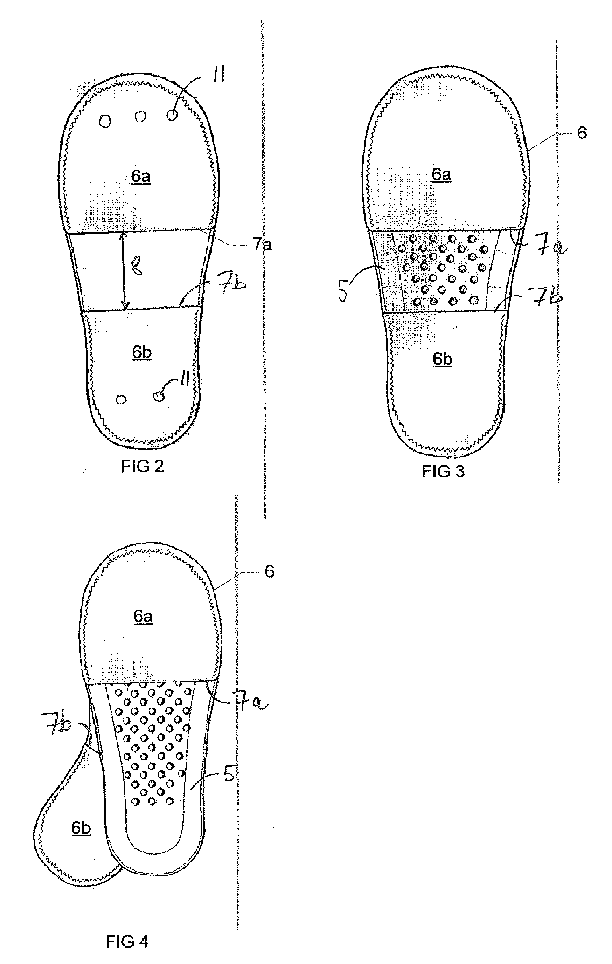

An insole 5 is secured under the foot in with the help of holding means to be described in detail with reference to figures 2 to 8. The insole 5 is provided in an elastic cushioning material and may have a thickness for example in the range of 5 - 20 millimetres depending on the patient and intended treatment.

Figures 2-4 show an embodiment of the holding means in the form of a compartment 6 for securing the insole 5. The compartment 6 comprises a front pocket 6a and a rear pocket 6b. The front pocket 6a has a front opening 7a directed to the rear, and the rear pocket 7b has a rear opening 7b directed to the front. The openings are separated by a distance 8. A distance in the range of 0-10 cm is suitable.

As seen in figure 3, the insole 5 is held securely in place in the two pockets 6a and 6b. The insole 5 should not be able to slide relative to the legging and the compartment 6 prevents it from moving in either direction, since the openings 7a and 7b are directed opposite one another.

The insole 5 is inserted in the compartment 6 by threading the insole first in the front pocket 6a, as shown in figure 4, and then in the rear pocket 6b, or vice versa. The removal of the insole 5 may be performed in the opposite order. The compartment is made of stretchable material to facilitate these operations. The compartment is preferably made of a material stretchable in two directions, such as Lycra.

The insole 5 is usually slightly cup-shaped at the heel part for accommodating the heel of the foot. Thus the rear pocket 6b should terminate a distance up on the heel of the legging 1. In this way, the seam of the compartment (not shown) is also placed further away from the area with foot pressure under the foot.

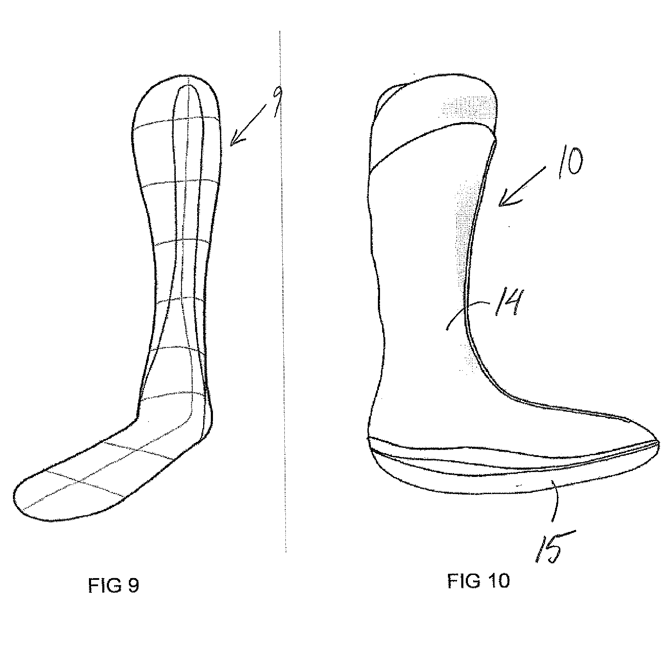

The relative sizes of the front pocket 6a and rear pocket 6b are not critical. It is preferred that the front pocket is larger than the rear pocket, such that an Achilles part 9, shown in figure 9, may also be inserted from the rear into the front pocket 6a.

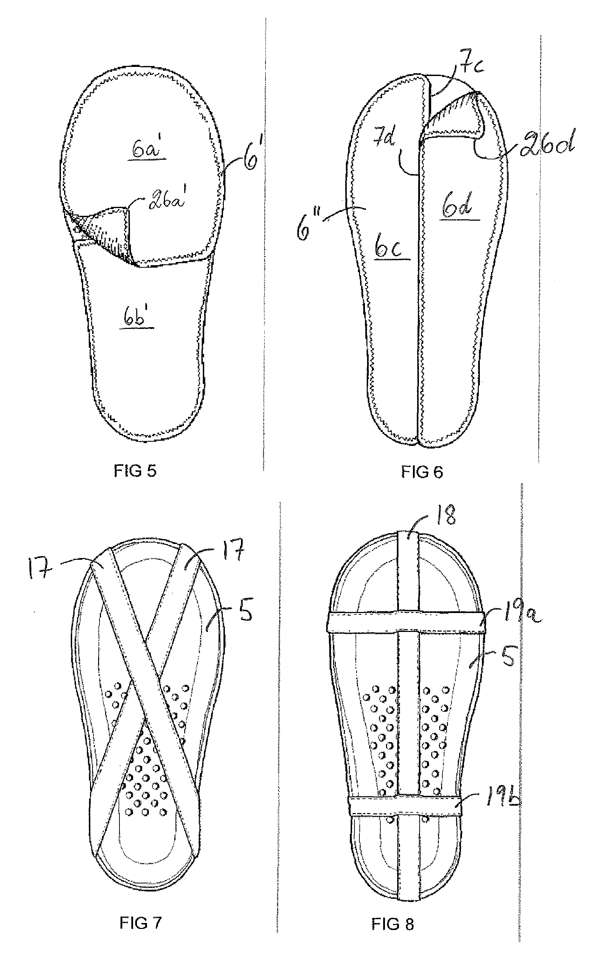

Also, the distance 8 between the openings may vary, and the front and rear pockets may even overlap, as shown in figure 5. The overlap may be in the range of 1 -2 cm. The overlap is illustrated with a flap 26a', but in the finished product the front pocket 6a is sewn all the way to the opening overlapping the rear pocket 6b. Similarly to figure 4, the insole is inserted in the compartment 6' by threading the insole first in the front pocket 6a', and then in the rear pocket 6b', or vice versa. With larger pockets, the insole is held more securely.

Figure 6 shows a further embodiment of the compartment 6" comprising two side pockets 6c and 6d with opposing longitudinal openings 7c and 7d, respectively. The overlap is illustrated with a flap 26d but in the finished product the front pocket 6a is sewn all the way to the toe end overlapping the side pocket 6c. Similarly to figure 4, the insole is inserted in the compartment 6" by threading the insole first in the side pocket 6d, and then in the side pocket 6c.

Figure 7 shows a further embodiment of the holding means comprising a pair of diagonal straps 17. The straps are attached at the front and rear side of the legging to prevent the insole 5 from slipping in any direction. The insole 5 is inserted under the straps 17, for example by threading the insole from one of the sides. An Achilles part 9, shown in figure 9, may also be inserted from the rear under the straps 17.

Figure 8 shows a further embodiment of the holding means comprising a longitudinal strap 18 and at least one, preferably a pair of transverse straps 19a, 19b. The strap 18 is attached at the front and rear side of the legging and the pair of transverse straps 19a, 19b at the sides of the legging a distance from the front and rear sides, respectively. The straps 18, 19a, 19b prevent the insole 5 from slipping in any direction. The insole 5 is inserted under the straps 18, 19a, 19b, for example by threading the insole from one of the sides.

In one embodiment the compartment 6 is provided with rubber pads 11 (fig. 2) or other friction materials suitably arranged for preventing slipping on the floor when the patient is walking.

For further protecting the leg and the foot, an Achilles part may be attached to the legging. An embodiment of an Achilles part 9 is shown in figure 9. The Achilles part 9 is designed to cover the underside of the foot, the heel, and Achilles tendon merging into the calf muscle. The Achilles part 9 may be attached to the legging 1 by means of straps and Velcro padding.

For best function and protection the legging 1 is further inserted in an outer shell 10 suitably manufactured of hard plastic material. Figure 10 shows an embodiment of an appliance comprising the legging inside a shell 10. The appliance comprises the legging, the shell 10, an outer textile 14, the insole, the Achilles part and a sole structure 15.

While particular embodiments of the present invention have been illustrated and described for purposes of disclosure, it will be obvious to a person skilled in the art that various changes and modifications can be made with regard to material and shape etc. The scope of the invention is only limited by the claims below.

## Claims

1. A legging (1) for a leg and foot, the legging (1) being adapted to substantially cover the leg and foot, **characterised by** a holding means (6, 6', 6", 17, 18, 19a, 19b) for securing a support member (5) at the underside of the foot.

2. A legging according to claim 1, wherein the holding means (6) comprises a front pocket (6a) and a rear pocket (6b).

3. A legging according to claim 2, wherein the front pocket (6a) comprises a front opening (7a) direct to the rear, and the rear pocket (6b) comprises a rear opening (7b) direct to the front.

4. A legging according to claim 3, wherein the front opening (7a) and the rear opening (7b) are separated by a distance (8).

5. A legging according to claim 4, wherein the distance (8) is in the range of 0 - 10 cm.

6. A legging according to any one of the preceding claims 2 to 5, wherein an Achilles part (9) is attached to the legging (1) and accommodated in the front pocket (6a).

7. A legging according to claim 3, wherein the front pocket (6a) overlaps the rear pocket (6b).

8. A legging according to claim 1, wherein the holding means (6") comprises two side pockets (6c) and (6d) with opposing longitudinal openings (7c) and (7d), respectively.

9. A legging according to claim 8, wherein the side pocket (6d) overlaps the side pocket (6c).

10. A legging according to claim 1, wherein the holding means comprises a pair of diagonal straps (17) attached at the front and rear side of the legging (1).

11. A legging according to claim 1, wherein the holding means comprises a longitudinal strap (18) and at least one transverse strap (19a, 19b), the strap (18) being attached at the front and rear side of the legging (1) and the transverse strap (19a, 19b) being attached at the sides of the legging (1) a distance from the front and rear sides.

12. A legging according to any one of the preceding claims, wherein the holding means (6, 6', 6", 17, 18, 19a, 19b) is made of stretchable material.

13. A legging according to any one of the preceding claims, wherein the legging (1) essentially is manufactured of a stretchable material.

14. A legging according to claim 13, wherein the stretchable material of the legging is a 3D spacer material.

15. A legging according to claim 14, wherein the stretchable material of the legging comprises polyester yarns knitted together with elastane.

16. A legging according to any one of the preceding claims, wherein the legging further is inserted in an outer shell (10).
